# EUROPEAN PATENT APPLICATION

(11) **EP 2 441 432 A1**
(43) Date of publication of application: **18.04.2012**
(21) Application number: 10187383.4
(22) Date of filing: 13.10.2010
(51) Int. Cl.: A61J 11/00, A61K 9/00

(54) **A suction device with external deposit of probiotics**

(71) Applicant: Nestec S.A., 1800 Vevey (CH)
(72) Inventor: Huber-Haag, Karl-Josef, 1009, Pully (CH); Bureau-Franz, Isabelle, 1066, Epalinges (CH)
(74) Representative: Corticchiato, Olivier

(57) **Abstract**

The invention relates to a suction device (1, 10, 20, 30) comprising a suction portion (3, 13, 23, 33) to be sucked by a user's mouth, characterized in that probiotics (7, 17, 27, 37) are adhered to or immobilized on the outer surface of the suction device (1, 10, 20, 30) such that the probiotics (7, 17, 27, 37) are segregated from the outer surface by external factors when the suction device (1, 10, 20, 30) is sucked.

## Description

### Field of the ivention

The present invention relates to a suction device, particularly a pacifier or a hollow feeding device such as a teat, comprising a suction portion, to a feeding apparatus comprising a container and said suction device, and to a method for making said suction device.

### Background

Straws or nipples, a part of which coming into contact with the user's mouth being impregnated or absorbed with odors or flavors, are known in the prior art as, for instance, in US 5,932,262.

WO 97/37636 shows an antibody being placed in the form of a liquid, emulsion or cream on or in a nipple. On the nipple there are paths, incisions or semi-permeable surfaces through which said antibody can seep into the mouth when the nipple is sucked. On the one hand, due to this arrangement, antibodies or the like may at least partially stick to the delivery system comprising the respective paths, incisions or semi-permeable surfaces such that not all the additives can reach the user's mouth. On the other hand, since a fluid additive (liquid, emulsion, cream) is needed to be used, a loss of said additive via the mentioned paths, incisions or semi-permeable surface may occur before use. Hence, it is difficult to determine the required amount of the antibody and additive.

In addition, the amount of the additives (like antibodies in the case of WO 97/37636) reaching the user's mouth is dependent on the suction power of the user. In the first months or years of their life, infant's suction power strongly increases and, in addition, all babies of the same age also have different suction powers. Therefore, the dosage of the additive cannot be certainly determined and, since in most cases not all the additive will be removed from the nipple, there will be a loss of additive, while in turn a bulk delivery of the additive still occurs.

US 6,203,566 B1 shows a pacifying or feeding dummy comprising an actual mouthpiece and a cover piece for attaching the dummy to a feeding bottle. The actual mouthpiece comprises a piece made of an elastic material to be held in the mouth of a baby between the tongue and the palate. The mouthpiece has a space formed into it for the insertion of a tablet or a comparable dosage unit containing an anti-caries agent or an agent against another disease. The tablet is inserted through an orifice being narrow as compared to the tablet. It is thus difficult to provide the dummy with the active agent as the orifice is comparably small with respect to the tablet to be inserted. Moreover, there are just provided little holes for the agent to be delivered into the mouth as a result of dissolution, erosion or its disintegration. Interaction with saliva or nutrition is thus negligible and the suction power is important for the release rate of the dosage and the duration of the release.

The present invention has been achieved in view of the above-mentioned drawbacks, and an object thereof is to improve the dilution of a probiotic deposit from the nipple directly to the mouth or body of a user in a safe, easy and efficient way.

There is a need for a device enabling and promoting the absorption of a probiotics thru a suckling device.

There is a need for such device where the contact of (a) the absorbed composition suckled out of the device and (b) the probiotic occurs in user's mouth.

There is a need for a device that promotes the correct dosage of the probiotics and absorption by the user.

There is a need to manufacture said device in a safe, simple and cost efficient way.

The object is to be accomplished by means of the independent claims. The dependent claims advantageously study further the central idea of the invention.

### Summary of the invention

According to an aspect of the invention, there is provided a suction device comprising a suction portion to be sucked by a user's mouth. The suction device is characterized in that probiotics are adhered to and/or immobilized on the outer surface of the suction device such that the probiotics are segregated from the outer surface by external factors when the suction device is sucked.

### Detailled description of the invention

By means of the above described feature, the probiotics are purposely located (adhered, immobilized, linked) by the manufacturer/producer at a place which is influenced by the suction of the user, particularly the outer surface of the suction portion. Hence, a segregation of the probiotics during a suction process can be securely accomplished and the absorption of a probiotics thru a suckling device is thus achieved. As the probiotics are located on an outer surface of the suction device, particularly on an outer surface of a suction portion, a complete removal can be insured due to, e.g., mechanical segregation, dilution and the like, as the probiotics are adhered or immobilized in a region which is influenced by the user's suction activity by means of deformation, friction and/or interaction with saliva or nutrition.

For a proper segregation the probiotics can be adhered to and/or immobilized on the outer surface of the suction device such that the probiotics are mechanically segregated from the outer surface when the user's mouth sucks the suction device and/or segregated by dissolution with water components or enzymes of the user's saliva or a nutrition. Alternatively or additionally, in case the suction device is preferably at least partially deformable, preferably at least the suction portion of the suction device, the probiotics can be adhered to and/or immobilized on the outer surface of the suction device such that the probiotics are mechanically segregated from the outer surface when the suction device is deformed.

The probiotics can thus directly be released in the user's mouth by the sucking action of the user, which leads to a deformation of the suction device by means of which the probiotics fall off or release from the outer surface and/or by (frictional) interaction with the users mouth enclosing the suction device and/or the user's saliva wherein its enzymes and/or water components cause dilution of the probiotics directly in the user's mouth. Hence, the supply of the probiotics, particularly the amount of which, is not (only) dependent on the suction power of the user since there is a fine balance between the adhesion/immobilization of the probiotics to/on the outer surface of the suction device and the ability to be released upon mechanical movement by sucking (due to the deformation of the suction device and/or the direct mechanical interaction with the user's mouth), the interaction with the saliva (or nutrition) and/or the temperature, for example. The probiotics thus completely fall off of the wall and/or are diluted by the saliva (or nutrition) directly in the user's mouth when the suction device is sucked and saliva (or nutrition) is thus getting into contact with the probiotics. The dosage of the probiotics can be exactly determined, e.g. to a controlled monodose, thus allowing to precisely insure a safe and efficient dosage of the probiotics and absorption by the user in comparison with a bulk delivery thereof.

In case the suction device or at least the suction portion of the suction device is deformable, the probiotics, when being placed in said deformable portion of the suction device, i.e. the suction portion, is securely segregated or released from the outer surface of the suction device since the segregation is promoted by the respective deformation of the suction device caused by the sucking action which in any case occurs when being used.

To summarize, by applying the probiotics deposition on the outer surface of the suction device or teat or pacifier, the saliva of the user, while having the suction device in the mouth, will insure the dilution of the probiotics directly in the mouth and exerts its beneficial effect. Moreover, by locating the probiotics deposit on an outside of the suction device, 10% to 80% of the suction device surface is covered that will be later in the user's mouth, thus a better mechanical effect of the release is accomplished. Moreover, the segregation of the probiotics works independent from the composition inside a bottle when a teat provided on a bottle is used. However, a (liquid) nutrition can also promote the dilution of the probiotics (or a matrix) when entering the user's mouth and getting into contact with the probiotics on the outer surface of the suction device. Additionally, the suction device can be easily manufactured by spraying or dipping it or at least its suction portion into a solution of probiotics (and a matrix).

Preferably, the outer surface of the suction device is treated to enable adhesion of the probiotics. Thereby, the probiotics can be easier adhered to or immobilized on the outer surface. The surface treatment can be done by roughening the surface. Such treatment can also include the coating with material comprising an oily component and/or a carbohydrate component and/or a proteinaceous component.

Preferably, the probiotics are mixed with a substance for promoting the adhesion or immobilization of the mix to/on the outer surface of the suction device and/or enhancing its stability. The substance can be a matrix, preferably comprising an oil (e.g. containing MCT), an emulsion, a gel or wax and/or can comprise carbohydrates or can be based on or derived from carbohydrates. The substance or matrix can also comprise proteinaceous materials or proteins. The substance, e.g., an oil or wax matrix, crystallizes at a temperature above room temperature (e.g. 40 degrees Celsius, preferably below body temperature) and is thus solid at room temperature. Hence, the probiotics can be easily applied to the outer surface of the suction device with a fluid matrix as a film cover which, after being applied thereto, cools down and gets solid, thus immobilizing the probiotics in the matrix. The probiotics can thus be securely adhered to the outer surface of the suction device and immobilized thereon. Moreover, by suction of the user (e.g. baby), the shape of the suction device, provided that it is deformable, is modified and due to this, the matrix-and-probiotics film breaks and falls off the outer surface directly into the user's mouth. Moreover, the probiotics are immobilized in the matrix which preferably releases the probiotics upon interaction with the saliva of the baby, especially the enzymes and/or the water components in the saliva. Said enzymes promote the dissolution of the substance or matrix by breaking bonds in said substance or matrix. Since the outer surface is at least partially enclosed by the user's mouth, a secure release of the probiotics can be accomplished by mechanical segregation as well.

Moreover, in a case in which the user's warm mouth encloses the suction device or a warm nutrition is fed, the matrix will solubilize by means of the body temperature or the nutrition passing the teat thus heating the suction device. Therefore, the body and/or the nutrition should have a temperature above the crystallizing temperature of the matrix-and-probiotics film. The probiotics can thus be easily displaced by the body temperature or nutrition due to its temperature and mechanical segregation.

In addition or alternative to the temperature, the release-ability of the matrix from the surface of the suction device can also be attained or enhanced by the pH and/or the salinity of the saliva or the nutrition which has already entered the mouth and gets into contact with the probiotics on the outer surface of the suction device. In other words, the segregation of the probiotics can be enhanced by either a dissolution of the probiotics and/or of the protective matrix (e.g. oil matrix or wax matrix), or a specific interaction in particular conditions such as pH of the saliva or nutritional composition enhancing segregation by acting on the protective matrix, and also by mechanical segregation via friction or deformation of the suction device or at least the suction portion when sucking the suction portion of the suction device. The segregation of the probiotics can thus be enhanced by the contact, during suckling, of the nutrition and the probiotics and/or said substance and/or said matrix.

In case of a teat as the suction device, the contact of the absorbed composition suckled out of the device and the probiotics occurs in the user's mouth to insure safe preservation for the probiotics when they are not stable in presence of the composition, and to enable the contacting at user's mouth temperature when probiotics may be instabled at the relatively higher temperature of the composition in the bottle.

Hence, the deposition properties of the probiotics can be easily determined and adjusted by varying the balance between immobilization of the probiotics in the matrix, the stickiness of said matrix to the outer walls of the suction device, and the ability of the matrix to be displaced by the saliva and/or nutrition and/or by the mechanical effect of the suction process, i.e., deformation or friction or the like. The mechanical and fluid interactions enable the full release of the probiotics, hence a correct dosage can be easier and better determined and thus absorbed by the user.

Preferably more than 10%, preferably more than 25%, preferably more than 50%, preferably more than 75%, preferably more than 90% of the outer surface of said suction device is covered by said probiotics and/or substance and/or matrix. Hence, the suction device is covered with said probiotics/ substance/ matrix and the more of the outer surface is covered the better the dissolution as the area of contact with the mouth is thus larger.

Preferably, the suction device is made of a flexible material, preferably a flexible polymeric material, more preferably silicon. By means of said feature, the suction device can be easily and economically produced while at the same time leads to a sufficient flexibility or formability such that the matrix-and-probiotics film can be easily segregated (i.e. broken) when the user sucks at the suction device, thus promoting the removal of the probiotics during the suction action.

The suction device can be a teat or a pacifier. Preferably, the suction device has a hollow form and further comprises an inlet portion having an inlet for entering nutrition, and the suction portion is designed for sucking the nutrition through the inlet into the suction device, wherein the suction portion comprises at least one opening for dispensing the sucked nutrition, wherein an outer surface of the suction device confines a flow path for the nutrition. By means of such an arrangement, i.e. in case the suction device is a feeding teat, nutrition flows through the suction device into the user's mouth. In case the probiotics are adhered to or immobilized on the outer surface of the suction device by a matrix, a warm nutrition having a temperature above the crystallizing temperature of the matrix-and-probiotics film can solubilize the matrix by means of the nutrition passing the teat thus heating the suction device. The probiotics can thus be easily displaced by the nutrition due to its temperature. Alternatively or additionally, the nutrition can also enter the user's mouth and can get into contact with the adhered (and immobilized) probiotics to dilute them from the outer surface of the suction device when being sucked by a user.

The suction device can preferably be enclosed in a protective shell, the protective shell preferably being air-tight and/or substantially oxygene tight and/or water tight, to isolate said suction device and protect the probiotics from degradation. The protective shell preferably comprises an aluminum foil or a polymeric material or a film or a combination thereof

According to another aspect of the invention, there is provided a feeding apparatus comprising: a container having an outlet, and a suction device according to the suction device described above. The suction device is mounted on the outlet of the container with its inlet portion. Hence, the suction device can be used in a commonly known apparatus as, for example, a baby bottle which can also be used for feeding animals or the like.

Preferably, the feeding apparatus further comprises a fixing means for removably linking the suction device to the container. The fixing means can be an adaptation ring with a closure mechanism or a screw thread. Hence, the suction device can be easily applied to and removed from the apparatus.

Further features, advantages and objects of the present invention would come apparent for the skilled person when reading the following detailed description of embodiments of the present invention, when taking in conjunction with the figures of the enclosed drawings.
- Fig. 1: shows a first embodiment of a suction device.
- Fig. 2: shows a second embodiment of a suction device.
- Fig. 3: shows a feeding apparatus comprising a third embodiment of a suction device.
- Fig. 4: shows a fourth embodiment of a suction device.

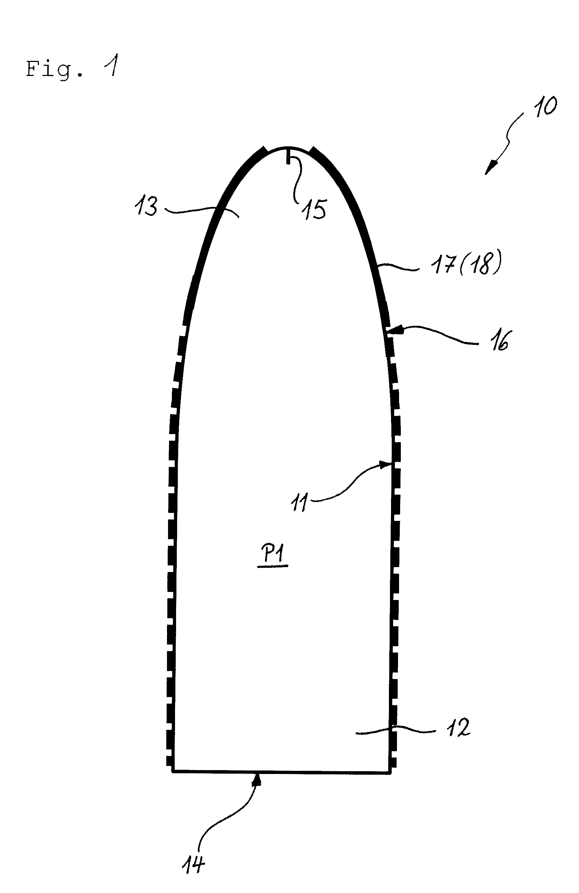

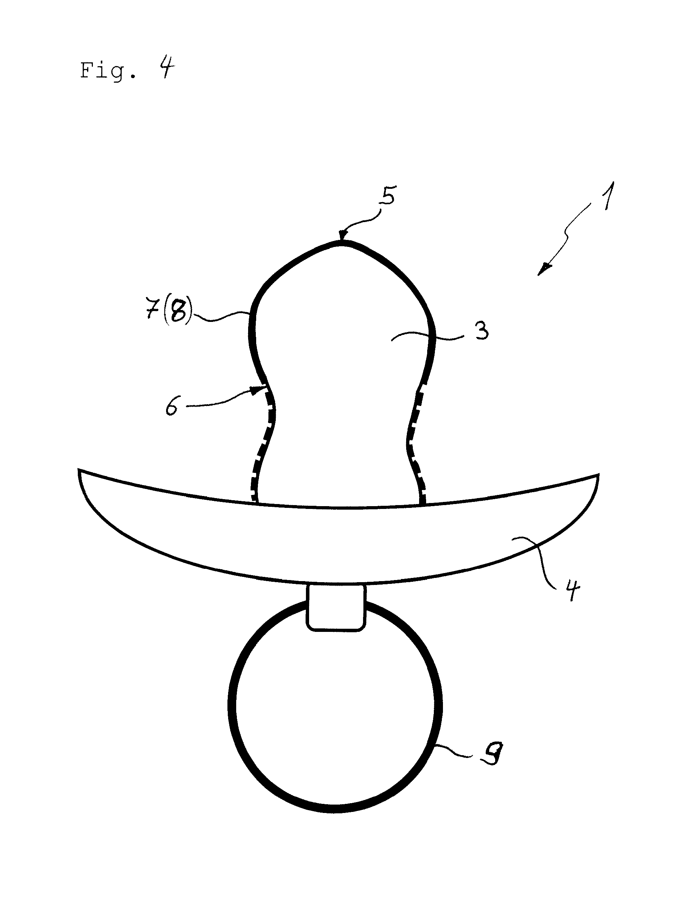

Figures 1 to 3 show a first to a third embodiment of a suction device 10, 20, 30 according to the invention according to which the suction device 10, 20, 30 is a (feeding) teat. Figure 4 shows a fourth embodiment of a suction device 1 according to the invention according to which the suction device 1 is a pacifier.

With respect to all the figures 1 to 4 according to the first to fourth embodiment, the suction device 1, 10, 20, 30 comprises a suction portion 3, 13, 23, 33 to be sucked by a user's mouth. The suction device 1, 10, 20, 30 can at least be partially deformable, preferably at least the suction portion 3, 13, 23, 33 is deformable, and can be made of any known material for suction devices, preferably a flexible material, more preferably a flexible polymeric material, further preferably silicon. However, the suction portion can also be made of an undeformable material.

According to the invention, probiotics 7, 17, 27, 37 are adhered to an outer surface 6, 16, 26, 36 of the suction device 1, 10, 20, 30. Therefore, the probiotics 7, 17, 27, 37 are purposely applied to said outer surface 6, 16, 26, 36.

Probiotic micro-organisms are micro-organisms which beneficially affect a host by improving its intestinal microbial balance. According to the currently adopted definition by FAO/WHO, probiotics are: "Live microorganisms which when administered in adequate amounts confer a health benefit on the host". In general, probiotic micro-organisms produce organic acids such as lactic acid and acetic acid which inhibit or influence the growth and/or metabolism of pathogenic bacteria such as Clostridium perfringens and Helicobacter pylori in the intestinal tract. Consequently, probiotic bacteria are believed to be useful in the treatment and prevention of conditions caused by pathogenic bacteria. Further, probiotic micro-organisms are believed to inhibit the growth and activity of putrefying bacteria and hence the production of toxic amine compounds. It is also believed that probiotic bacteria activate the immune function of the host.

Examples of suitable probiotic micro-organisms include yeasts such as Saccharomyces, Debaromyces, Candida, Pichia and Torulopsis, moulds such as Aspergillus, Rhizopus, Mucor, and Penicillium and Torulopsis and bacteria such as the genera Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus and Lactobacillus. Specific examples of suitable probiotic micro-organisms are: Saccharomyces cereviseae, Bacillus coagulans, Bacillus licheniformis, Bacillus subtilis, Bifidobacterium bifidum, Bifidobacterium infantis, Bifidobacterium longum, Enterococcus faecium, Enterococcusfaecalis, Lactobacillus acidophilus, Lactobacillus alimentarius, Lactobacillus casei subsp. casei, Lactobacillus casei Shirota, Lactobacillus curvatus, Lactobacillus delbruckii subsp. lactis, Lactobacil- 2Q lus farciminus, Lactobacillus gasseri, Lactobacillus helveticus, Lactobacillus johnsonii, Lactobacillus reuteri, Lactobacillus rhamnosus (Lactobacillus GG), Lactobacillus sake, Lactococcus lactis, Micrococcus varians, Pediococcus acidilactici, Pediococcus pentosaceus, Pediococcus 25 acidilactici, Pediococcus halophilus, Streptococcusfaecalis, Streptococcus thermophilus, Staphylococcus carnosus, and Staphylococcus xylosus.

The probiotic bacteria may be used live, inactivated or dead or even be present as fragments such as DNA or cell wall materials. In other words, the quantity of bacteria which the formula contains is expressed in terms of the equivalent colony forming units of bacteria irrespective of whether they are, all or partly, live, inactivated, dead or fragmented.

The probiotic bacterial strain may be any lactic acid bacteria or Bifidobacteria with established probiotic characteristics. The probiotic of the invention may be any probiotic bacteria or probiotic microorganism that have been or can be originated, found, extracted or isolated in milk upon excretion, preferably in human breast milk. Suitable probiotic lactic acid bacteria include Lactobacillus rhamnosus ATCC 53103 obtainable inter alia from Valio Oy of Finland under the trade mark LGG, Lactobacillus rhamnosus CGMCC 1.3724, Lactobacillus reuteri ATCC 55730 and Lactobacillus reuteri DSM 17938 obtainable from Biogaia, Lactobacillus fermentum VRI 003 and Lactobacillus paracasei CNCM I-2116, Lactobacillus johnsonii CNCM I-1225, Lactobacillus Helveticas CNCM I-4095, Bifidobacterium breve CNCM I-3865, Bifidobacterium longum CNCM I-2618.

Suitable probiotic Bifidobacteria strains include Bifidobacterium longum ATCC BAA-999 sold by Morinaga Milk Industry Co. Ltd. of Japan under the trade mark BB536, the strain of Bifidobacterium breve sold by Danisco under the trade mark Bb-03, the strain of Bifidobacterium breve sold by Morinaga under the trade mark M-16V and the strain of Bifidobacterium breve sold by Institut Rosell (Lallemand) under the trade mark R0070. A particularly preferred Bifidobacterium strain is Bifidobacterium lactis CNCM I-3446 which may be obtained from the Christian Hansen Company of Denmark under the trade mark Bb12. A mixture of suitable probiotic lactic acid bacteria and Bifidobacteria may be used.

In the following, the suction device according to figure 1 will be described in more detail.

The suction device 10 is hollow, thus defining an inner surface 11. The inner surface 11 defines or confines a flow path P1 for nutrition, such that nutrition can pass through the suction device 10. The suction device 10 comprises an inlet portion 12 and a suction portion 13. The suction device 10 can also be at least partially deformable or undeformable.

The inlet portion 12 can comprises an inlet 14 for entering nutrition when the suction device 10 is sucked. Therefore, the suction device 10 can be used as a straw thus sucking the nutrition via the inlet 14 into the suction device 10 or, preferably, as a nipple, e.g. for a baby bottle, as also described later.

The nutrition can be any kind of nutrition which is eaten or drunken by the use of such a suction device 10. Preferably, the nutrition is a liquid nutrition, but the invention is not limited thereto.

The suction portion 13 comprises a suction and outlet opening 15, in the following also referred to as opening. By means of said opening 15, a user can suck the nutrition through the inlet 14 of the inlet portion 12 into the suction device 10. The sucked nutrition can then be dispensed through said opening 15. In a preferred embodiment, the opening 15 in the suction portion 13 is a valve means being designed such that it only opens under suction. Thereby, a loss of nutrition can be avoided when the suction device is not in use, i.e. not sucked nor deformed.

The inner surface 11 confining said flow path P1 preferably extends from the inlet 14 to the opening 15.

The suction device 10 may also comprises at least a further opening (not shown). By means of said feature, the flow of air through said hole or opening can be enabled during suction when the suction device 10 is adapted to a non-flexible container, e.g. a glass container, or the like, but may also facilitate the sucking action when using a flexible container or no container at all. Hence, said opening is used as an air inlet when the suction device 10 is sucked for attaining a pressure compensation.

At an outer surface 16 of the suction device 10, i.e. a suction outer surface 16, probiotics 17 are adhered. Therefore, the probiotics 17 are purposely applied to said outer surface 16.

The suction device 1 according to the fourth embodiment (see figure 4) can further comprise a mouth shield 4 and a handle 9 as commonly known in the prior art.

With respect to both the before-mentioned embodiments (i.e. first and fourth embodiment) it has been described that the probiotics 7, 17 are purposely adhered to the outer surface 6, 16 of the suction device 1, 10, preferably at least to the outer surface of the suction portion 3, 13. As the probiotics 7, 17 are thus also necessarily located by the manufacturer/producer at a region being influenced by the user when using the suction device 1, 10, i.e. when being influenced by the sucking action of the user, the probiotics 7, 17 are segregated from the outer surface 6, 16 by external factors when the suction device 1, 10 is sucked.

Hence, a segregation of the probiotics 7, 17 during a suction process can be securely accomplished. As the probiotics 7, 17 are located on an outer surface 6, 16 of the suction device 1, 10, particularly on an outer surface of a suction portion 3, 13, a complete removal can be insured due to, e.g., mechanical segregation, dilution and the like, as the probiotics 7, 17 are adhered or immobilized in a region which is influenced by the user's suction activity by means of deformation, friction and/or interaction with saliva or nutrition as will be described in detail in the following.

For a proper segregation the probiotics 7, 17 can be adhered to or immobilized on the outer surface 6, 16 of the suction device 1, 10 such that the probiotics 7, 17 are mechanically segregated from the outer surface 6, 16 when the user's mouth sucks the suction device 1, 10 and/or segregated by dissolution with water components or enzymes of the user's saliva or a nutrition. Alternatively or additionally, in case the suction device 1, 10 is preferably at least partially deformable, preferably at least the suction portion 3, 13 of the suction device 1, 10, the probiotics 7, 17 can be mechanically segregated from the outer surface 6, 16 when the suction device 1, 10 is deformed.

The probiotics 7, 17 can thus directly be released in the user's mouth by the suction of the user, which leads to a deformation of the suction device 1, 10 by means of which the probiotics 7, 17 fall off or release from the outer surface 6, 16 and/or by (frictional) interaction with the users mouth enclosing the suction device 1, 10 and/or the user's saliva wherein its enzymes and/or water components cause dilution of the probiotics 7, 17 directly in the user's mouth. Hence, the supply of the probiotics 7, 17, particularly the amount of which, is not (only) dependent on the suction power of the user since there is a fine balance between the adhesion of the probiotics 7, 17 to the outer surface 6, 16 of the suction device 1, 10 and the ability to be released upon mechanical movement by sucking (due to the deformation of the suction device 1, 10 and/or the direct mechanical interaction with the user's mouth), the interaction with the saliva (or nutrition) and/or the temperature, for example. The probiotics 7, 17 thus completely fall off of the wall and/or are diluted by the saliva (or nutrition) directly in the user's mouth when the suction device 1, 10 is sucked and saliva (or nutrition) is thus getting into contact with the probiotics 7, 17. The dosage of the probiotics can be exactly determined, e.g. to a controlled monodose, thus allowing to precisely insure a safe and efficient dosage of the probiotics in comparison with a bulk delivery thereof.

In case the suction device 1, 10 or at least the suction portion 3, 13 of the suction device 1, 10 is deformable, the probiotics 7, 17, when being placed in said deformable portion of the suction device 1, 10, i.e. the suction portion 3, 13, is securely segregated or released from the outer surface 6, 16 of the suction device 1, 10 since the segregation is promoted by the respective deformation of the suction device 1, 10 caused by the sucking action which in any case occurs when being used.

Hence, by applying the probiotics deposition 7, 17 on the outer surface 6, 16 of the suction device 1, 10 or teat or pacifier, the saliva of the user, while having the suction device in the mouth, will insure the dilution of the probiotics 7, 17 directly in the mouth and exerts its beneficial effect. Moreover, by locating the probiotics deposit 7, 17 on an outside of the suction device 1, 10, 10% to 80% of the outer surface 6, 16 that will be later in the user's mouth is covered, thus a better mechanical effect of the release is accomplished. Moreover, the segregation of the probiotics 7, 17 works independent from the composition inside a bottle when a teat provided on a bottle is used. However, a (liquid) nutrition can also promote the dilution of the probiotics 7, 17 (or a matrix) when entering the user's mouth and getting into contact with the probiotics 7, 17 on the outer surface 6, 16 of the suction device 1, 10. Additionally, the suction device 1, 10 can be easily manufactured by spraying or dipping it or at least its suction portion 3, 13 into a solution of probiotics 7, 17 (and a matrix).

As described above, the mechanical segregation can be enhanced in case the suction device 1, 10 is at least partially deformable, preferably made of a flexible material, which can be a flexible polymeric material, preferably silicon. By using such a material, the suction device 1, 10 can be easily and economically produced while at the same time leading to a sufficient flexibility or formability such that the probiotics film or a matrix-and-probiotics film (which will be described lateron) can be easily broken due to mechanical segregation when the user sucks at the suction device 1, 10, thus promoting the removal of the probiotics 7, 17 during the sucking action.

As the probiotics 7, 17 are adhered to a region of the suction device 1, 10, i.e. its outer surface 6, 16 which is at least partially enclosed by the user's mouth when being sucked, the probiotics 7, 17 can be quickly diluted or segregated at the beginning of the suction process even if the nutrition, e.g. placed in a bottle or the like of a teat, is not fully finished or the pacifier is merely sucked for a short time. Hence, a controlled dosage applied to the user can be securely achieved even when applying only little suction power or having a short sucking duration.

To enhance the influence of the external factors, the probiotics 7, 17 are preferably adhered to or immobilized on a portion at the outer surface 6, 16 of the suction device 1, 10, which is securely enclosed by the user's mouth and which thus gets into contact with the user's mouth, tongue and saliva. In this regard, the solid line in Fig. 1 and 4 refers to a preferred positioning of the probiotics 7, 17. In this preferred embodiment, the probiotics 7, 17 are at least adhered to the outer surface 6, 16 of the suction portion 3, 13, in a more preferred embodiment at least close to the tip 5 or outlet opening 15 of the suction portion 3, 13. In this preferred and more preferred positioning of the probiotics 7, 17 at the outer surface 6, 16 of the suction device 1, 10, the influence of the external factors (e.g. dilution with saliva or deformation or friction) is maximized.

The dashed line in Fig. 1 and 4 refers to another possible positioning of the probiotics 7, 17. However, the invention is not limited to the before-mentioned positioning of the probiotics 7, 17. The probiotics 7, 17 can be applied to any position on the outer surface 6, 16 of the suction device 1, 10 as long as at least one of the before-mentioned external factors can have an influence on the probiotics 7, 17 for segregating them to directly enter the user's mouth.

The adhesion or immobilization of the probiotics 7, 17 to/on the outer surface 6, 16 of the suction device 1, 10 can be accomplished in a plurality of ways, which are described in the following.

The outer surface 6, 16 of the suction device 1, 10 can be treated to enable adhesion of the probiotics 7, 17. Thereby, the probiotics 7, 17 can be easier adhered to the outer surface 6, 16. The surface treatment can be done by roughening the surface 6, 16, for instance. However, any possibility known by the person skilled in the art for making a surface more adhesive-friendly is covered by the invention, which is thus not limited to the before-mentioned examples.

Additionally, the probiotics 7, 17 can also be mixed with a substance 8, 18 having a good stickiness for promoting the adhesion of the mix to the outer surface 6, 16 of the suction device 1, 10 and/or enhancing its stability. Such a substance 8, 18 can be a matrix, preferably comprising an oil (e.g. containing MCT), an emulsion, a gel or wax and/or can comprise carbohydrate or can be based on or derived from carbohydrates. The substance 8, 18 or matrix can also comprise proteinaceous materials or proteins. Any other known substances which are usually known by the person skilled in the art for such intended uses are also covered by the invention.

The substance 8, 18, e.g. an oil or wax matrix, preferably crystallizes at a temperature above room temperature (e.g. 40 degrees Celsius or preferably between room temperature and body temperature). Hence, the substance 8, 18 is solid at room temperature. For easily applying the probiotics 7, 17 to the outer surface 6, 16 of the suction device 1, 10, the probiotics 7, 17 are mixed with said substance 8, 18 or matrix being in a fluid condition. Then, the matrix-and-probiotics mixture can be easily applied to the outer surface 6, 16 by spray coating or the like to attain a film cover which, after being applied to said outer surface 6, 16, cools down and gets solid, thus immobilizing the probiotics 7, 17 in the matrix. The probiotics 7, 17 can thus be securely adhered to the outer surface 6, 16 of the suction device 1, 10. Even if the before-mentioned way of applying the matrix-and-probiotics mixture onto the outer surface 6, 16 of the suction device 1, 10 is preferred, the invention is not limited to thereto. Other ways of applying said mixture known by the person skilled in the art are also covered by the invention such as dipping the suction device 1, 10 in a liquid or matrix containing probiotics and air-dry said film cover or dry the film cover under nitrogene or by means of temperature or the like.

Again referring to Fig. 1 and 4, by suction of the user, the shape of the suction device 1, 10 can be modified, provided that it is deformable, and due to this, the matrix-and-probiotics film breaks and falls off the outer surface 6, 16 and subsequently falls into the user's mouth enclosing the suction device 1, 10.

In a case in which the user's warm mouth encloses the suction device or a warm nutrition (having a temperature above the crystallizing temperature of the matrix) is fed, the matrix solubilizes and thus liquefies again by means of the body temperature or the passing warm/hot nutrition thus heating the suction device and, due to thermal conduction, also heating the matrix. Therefore, the body and/or the nutrition having a temperature above the crystallizing temperature of the matrix-and-probiotics film. The matrix or probiotics can thus be easily displaced by the body temperature or the nutrition due to its temperature and preferably also by virtue of mechanical segregation.

In addition or alternative to the temperature, the release-ability of the matrix from the outer surface 6, 16 of the suction device 1, 10 can also be attained or enhanced by the pH and/or the salinity of the user's saliva or the nutrition which has already entered the user's mouth and gets into contact with the probiotics 7, 17 on the outer surface 6, 16 of the suction device 1, 10 or other factors commonly known by the person skilled in the art. In other words, the segregation of the probiotics 7, 17 can be enhanced by either a dissolution of the probiotics 7, 17 and/or of the protective matrix 8, 18 (e.g. oil matrix or wax matrix), or a specific interaction in particular conditions such as pH of the saliva or nutritional composition enhancing segregation by acting on or interacting with the protective matrix 8, 18, and also by mechanical segregation via friction or deformation of the suction device 1, 10 or at least the suction portion 3, 13 when sucking the suction portion 3, 13 of the suction device 1, 10. The segregation of the probiotics 7, 17 can thus be also enhanced by the contact, during suckling, of said nutrition and said probiotics 7, 17 and/or said substance 8, 18 and/or said matrix.

Hence, the deposition properties of the probiotics 7, 17 can be easily determined and adjusted by varying the balance between immobilization of the probiotics 7, 17 in the substance 8, 18, the stickiness of said substance 8, 18 or matrix to the walls (i.e. outer surface 6, 16) of the suction device 1, 10, and the ability of the substance 8, 18 or matrix to be displaced by the saliva and/or nutrition and/or by the mechanical effect of the suction process, i.e., deformation or friction or the like.

Preferably more than 10%, preferably more than 25%, preferably more than 50%, preferably more than 75%, preferably more than 90% of the outer surface of said suction device 1, 10 (or suction portion 3, 13) is covered by said probiotics 7, 17 and/or substance 8, 18 and/or matrix. Hence, the suction device 1, 10 is covered with said probiotics 7, 17 and/or substance 8, 18 and/or matrix and the more of the outer surface is covered the better the dissolution as the area of contact with the mouth is thus larger.

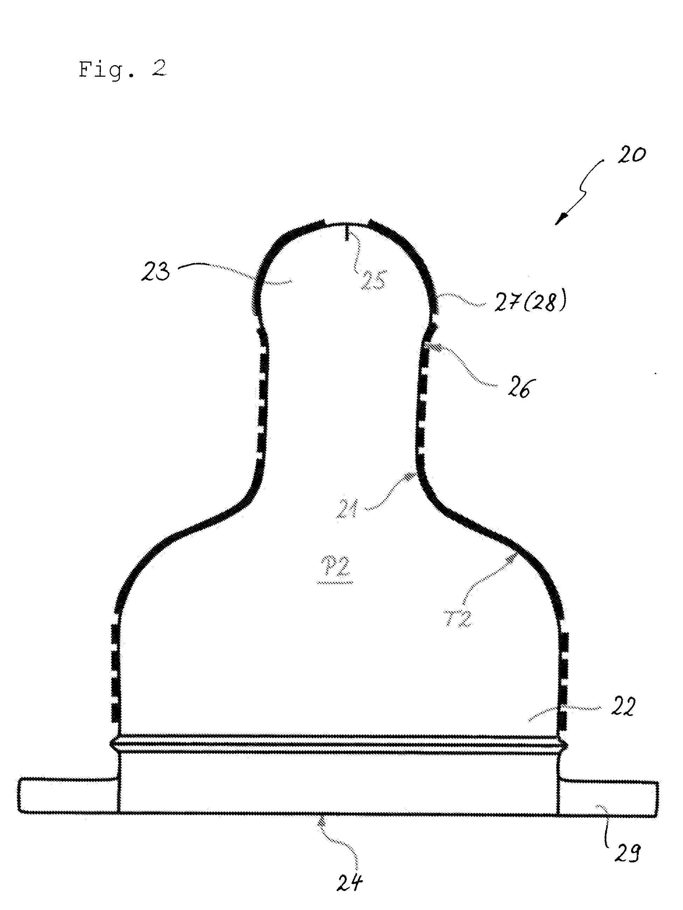

Figure 2 shows a second embodiment of a suction device 20 according to the present invention. The suction device 20 also comprises an inlet portion 22 with an inlet 24 and a suction portion 23 with an opening 25 as well as probiotics 27 (preferably being mixed with a substance 28) adhered to the outer surface 26. An inner surface 21 confines a flow path P2 of the suction device 20. The respective features of this embodiment have the same function and properties as the features mentioned in the before-mentioned embodiments, particularly the first embodiment. Everything which has been said about the first and fourth embodiment thus also applies mutatis mutandis to the second embodiment.

In addition to the suction device 10 according to the first embodiment, the suction device 20 of Fig. 2 has a more anatomical shape. This anatomical shape has at least two diameters, wherein the inlet portion 22 has a bigger diameter than the suction portion 23. The small diameter of the suction portion 23 enables the lips of a user to grab the suction portion 23 or nipple or teat, and the larger diameter of the inlet portion 22 preferably fits a diameter of a container (e.g. a bottle). The transition portion T2 extending from the small diameter to the larger diameter can act as a stopper for the user's mouth.

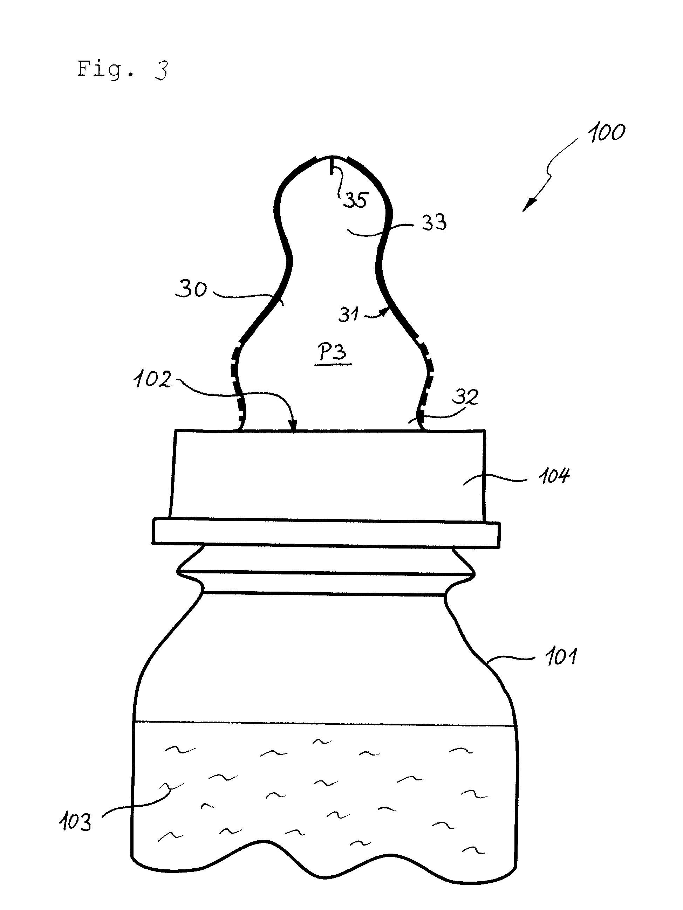

The shown suction device 20 also has an outwardly extending flange portion 29 at its lower end/bottom portion close to the inlet 24 of the inlet portion 22. Said flange portion 29 can serve as a support when mounting the suction device 20 to a container as will be described with reference to Fig. 3 below.

The suction device 20 thus has a commonly known shape of a nipple for a baby bottle. The invention, however, is not limited to this design or number of diameters or its dimensions.

As can be seen in Fig. 2, the probiotics 27 are preferably located at regions (solid lines) on an outside of the suction device 20 and on/at its outer surface 26, i.e. a portion being enclosed by the user's mouth when being used, where the influence of the external factors (e.g. dilution with saliva or deformation or friction) is enhanced or maximized. The probiotics 27, however, can be applied to any other region (e.g. dashed line) on the outer surface 26 of the suction device 20 as long as at least one of the before-mentioned external factors can have an influence on the probiotics 27 or matrix 28 for segregating them to directly enter the user's mouth.

Additionally, Fig. 3 shows a feeding apparatus 100 according to the invention comprising a container 101, and a suction device 30. The container 101 comprises an outlet 102 for dispensing the nutrition 103 being stored inside the container 101. Everything which has been said in respect with the suction devices 1, 10, 20 according to the first, second and fourth embodiments, particularly to the first and second embodiments, also applies mutatis mutandis for the suction device 30 shown in Fig. 3 having the same features with corresponding references.

The suction device 30 is mounted on the container 101 with its inlet portion 32 such that nutrition 103 being stored in the container 101 can exit the container 101 through its outlet 102 and enter the suction device 30 through its inlet (see inlet 24 in Fig. 2, for instance) when in use. Hence, a flow of the nutrition out of the container 101 into the suction device 30 and through said suction device 30 via the flow path P3 (confined by the inner surface 31) and then out of the suction device 30 via the opening 35 is provided, such that the suction device 30 can be used in a commonly known apparatus as, for example, a baby bottle which can also be used for feeding animals or the like.

In the preferred embodiment shown in Fig. 3, the feeding apparatus 100 preferably comprises a fixing means 104 for removably linking the suction device 30 to the container 101. The fixing means 104 can be an adaptation ring with a closure mechanism or a screw thread such that the suction device 30 can be easily applied to and removed from the container 101.

Preferably, the suction device 30 can be placed on the outlet 102 of the container 101 with its flange portion (cf. Fig. 2: 29), which then is pinched or clamped between the container 101, i.e. the outlet 102 of said container 101, and the fixing means 104 in a commonly known way, which is thus not further explained. In a preferred embodiment, the fixing means 104 is an adaptation ring with a closure mechanism or a screw thread. However, the suction device 30 can be mounted to the container 101 in any known way, preferably as long as a nutrition 103 flow from the container 101 into the suction device 30 and thus into the user's mouth is guaranteed and the connection is preferably sealed such that no nutrition 103 can leak. The suction device 30 can also be imposed on the outlet 102 of the container 101 without the use of any fixing means. If necessary, the imposed suction device 30 can also be fixed with the aid of a rubber band or clip or any other fixing means known by the person skilled in the art.

In the following, a suction process will be described.

In case of a pacifier as suction device 1 having probiotics or a matrix-and-probiotics mixture adhered to and/or immobilized on the outer surface 6 of the suction device 1 or suction portion 3, the user puts the suction device, i.e. at least the suction portion 6 thereof, into his/her mouth. Due to external factors, the probiotics 7 are segregated from the outer surface 6 when the suction device is sucked. Such external factors are a friction occurring because of the sucking action and/or a deformation of the suction device 1/suction portion 6 when sucking it and/or an interaction of the water component or enzyms of the user's saliva with the probiotics 7 or matrix 8 and/or an interaction of the nutrition which has entered the user's mouth with the probiotics 7 or matrix 8 and/or body temperature of the user or the temperature of the nutrition solubilizing the matrix-and-probiotics film 8 and/or other possible factors. Particularly, said enzymes of the user's saliva promote the dissolution of the substance or matrix by breaking bonds in said substance or matrix 8. Hence, the probiotics 7 which have been adhered to or immobilized on the outer surface 6 of the suction device 1 can directly enter the user's mouth when using it independent from the suction power and duration.

In case of a teat, the suction device 10, 20, 30 can be mounted, e.g. via a fixing means 104, on a container 101 comprising a nutrition 103, or can be used as a straw or the like for sucking a nutrition 103, e.g. stored in a receptacle or the like. Then, the user sucks at the suction portion 13, 23, 33 of the suction device 10, 20, 30. Due to the sucking action through the opening 15, 25, 35 in the suction portion 13, 23, 33, preferably low-pressure occurs in the suction device 10, 20, 30 thus nutrition 103 is sucked out of the container 101 or other receptacle or the like through the inlet 14, 24 of (or in) the inlet portion 12, 22, 32 into the suction device 10, 20, 30. Then, the nutrition 103 passes or flows through the suction device 10, 20, 30, i.e. the nutrition 103 flows through the flow path P1, P2, P3 confined by the inner surface 11, 21, 31 of the suction device 10, 20, 30, towards the suction portion 13, 23, 33. The sucked nutrition 103 is then dispensed through the opening 15, 25, 35 into the user's mouth.

The probiotics 17, 27, 37 are adhered to the outer surface 16, 26, 36 of the suction device 10, 20, 30 as described above, preferably supported by a substance 18, 28, 38 for promoting the adhesion of the substance-and-probiotics mixture to the outer surface 16, 26, 36 and/or enhancing its stability. The probiotics 17, 27, 37 adhered to said outer surface 16, 26, 36, i.e. a portion enclosed by the user's mouth when using the suction device, is then segregated by means of the external factors as already described in respect of the suction process of the pacifier. Then, probiotics 17, 27, 37 directly fall or enter the user's mouth independent from the nutrition, the suction power and duration.

In the following, a method for making the suction device 1, 10, 20, 30 or better for providing the suction device 1, 10, 20, 30 with probiotics 7, 17, 27, 37 (and/or a substance 8, 18, 28, 38 and/or a matrix) according to any one of the first to fourth embodiment will be described:

According to this method, the outer surface of said suction device 1, 10, 20, 30 is contacted with said probiotics 7, 17, 27, 37 and/or said substance 8, 18, 28, 38 and/or said matrix for adhering or immobilizing the probiotics 7, 17, 27, 37 to/on the outer surface of the suction device 1, 10, 20, 30 such that the probiotics 7, 17, 27, 37 are segregated from the outer surface by external factors when the suction device 1, 10, 20, 30 is sucked.

Optionally, an aquous portion of said probiotics 7, 17, 27, 37 and/or substance 8, 18, 28, 38 and/or matrix is evaporated on said outer surface of the suction device 1, 10, 20, 30 for promoting said adhesion or immobilization. The evaporated aquous portion of said probiotics 7, 17, 27, 37 and/or substance 8, 18, 28, 38 and/or matrix can then be dried actively (by heat supply or the like) or passively to attain the adhesion or immobilization of the probiotics 7, 17, 27, 37 to/on the outer surface of the suction device 1, 10, 20, 30.

Preferably, the contacting is made in the form of spraying and/or dipping and/or depositing said probiotics 7, 17, 27, 37 and/or substance 8, 18, 28, 38 and/or matrix onto said outer surface of the suction device 1, 10, 20, 30.

By means of the before-mentioned method for making the suction device 1, 10, 20, 30 or better for providing the suction device 1, 10, 20, 30 with probiotics 7, 17, 27, 37 (and/or a substance 8, 18, 28, 38 and/or a matrix), the suction device 1, 10, 20, 30 can be manufactured in a safe, simple and cost efficient way. The contacting (e.g. spraying or dipping or the like) of the probiotics 7, 17, 27, 37 is easier to be accomplished to the outside surface of the suction device 1, 10, 20, 30 in comparison with the probiotics being provided on an inner surface thereof or in pouches or incisions provided in the suction device. Hence, the suction device 1, 10, 20, 30 can be made or the probiotics 7, 17, 27, 37 and/or a substance 8, 18, 28, 38 and/or a matrix can be provided on ( i.e., adhered to or immobilized on) the outer surface of the suction device 1, 10, 20, 30 in a more economic way as less dispersion of the probiotics 7, 17, 27, 37 occurs. Additionally, the outer surface of the suction device 1, 10, 20, 30 is much easier to access on a manufacturing line such that the suction device 1, 10, 20, 30 can be easily manufactured or provided with probiotics 7, 17, 27, 37 and/or a substance 8, 18, 28, 38 and/or a matrix by way of an automated manufacturing process.

In a preferred embodiment, the suction devices 1, 10, 20, 30, supposedly provided with the probiotics 7, 17, 27, 37 by the manufacturer/producer, are individually packed in a protective shell and thus enclosed by a material promoting the preservation and stability of the probiotics 7, 17, 27, 37 to isolate said suction device and thus to protect the probiotics from degradation. Such a material can be, for instance, a polymeric or aluminum air-tight foil or a protective capsule for storage, preservation, and the like. Hence, the protective shell preferably comprises an aluminum foil or a polymeric material or a film or a combination thereof. Preferably, the suction device 1, 10, 20, 30 is packed in an aluminum blister and gassed. Thereby, a germ-free environment can be achieved to obtain the high purity and sanitization standards for such devices which enhances a bacterial protection. The foil or capsule can be moisture tight, water tight, air-tight, oxygen tight, and/or impervious to light. Nevertheless, the invention is not limited to the before-mentioned kinds of packaging.

Although the present invention has been described with reference to preferred embodiments thereof, many modifications and alternations may be made by a person having ordinary skill in the art without departing from the scope of this invention which is defined by the appended claims. For example, the use of the suction device is not limited to a baby bottle, but can be used as a suction device in any kind of feeding apparatuses known in the state of the art as, for instance, an apparatus for suckling animals or the like, or it can be used solely, e.g. as a straw, or it can be used as a pacifier. Moreover, the regions to which the probiotics are applied to or immobilized on the outer surface of the suction device is not limited by the invention. The shape and material of the suction device is also not limited as long as being covered by the subject-matter of the appended claims and the intended use.

### Reference numerals

- 1, 10, 20, 30: suction device
- 11, 21, 31: (flow path confining) inner surface of the suction device
- 12, 22, 32: inlet portion
- 3, 13, 23, 33: suction portion
- 4: shield
- 14, 24: inlet of (or in) the inlet portion (inlet)
- 5: tip of the suction portion
- 15, 15, 25: suction and outlet opening (opening)
- 6, 16, 26, 36: outer surface of the suction device
- 7, 17, 27, 37: probiotics
- 8, 18, 28, 38: substance (matrix)
- 9: handle
- 29: flange portion
- 100: feeding apparatus
- 101: container
- 102: outlet (outlet opening)
- 103: nutrition
- 104: fixing means
- P1, P2, P3: flow path
- T2: transition portion

## Claims

1. A suction device (1, 10, 20, 30) comprising a suction portion (3, 13, 23, 33) to be sucked by a user's mouth,
**characterized in that**
probiotics (7, 17, 27, 37) are adhered to or immobilized on the outer surface of the suction device (1, 10, 20, 30) such that the probiotics (7, 17, 27, 37) are segregated from the outer surface by external factors when the suction device (1, 10, 20, 30) is sucked.

2. The suction device (1, 10, 20, 30) according to claim 1,
**characterized in that**
wherein the probiotics (7, 17, 27, 37) are at least adhered to or immobilized on the outer surface of the suction portion (3, 13, 23, 33).

3. The suction device (1, 10, 20, 30) according to claim 1 or claim 2,
**characterized in that**
the probiotics (7, 17, 27, 37) are adhered to or immobilized on the outer surface of the suction device (1, 10, 20, 30) such that the probiotics (7, 17, 27, 37) are mechanically segregated from the outer surface when the user's mouth sucks the suction device.

4. The suction device (1, 10, 20, 30) according to any of the preceding claims,
**characterized in that**
the probiotics (7, 17, 27, 37) are adhered to or immobilized on the outer surface of the suction device (1, 10, 20, 30) such that the probiotics (7, 17, 27, 37) are segregated by dissolution with water components of the user's saliva.

5. The suction device (1, 10, 20, 30) according to any of the preceding claims,
**characterized in that**
the probiotics (7, 17, 27, 37) are adhered to or immobilized on the outer surface of the suction device (1, 10, 20, 30) such that the probiotics (7, 17, 27, 37) are segregated by dissolution with enzymes of the user's saliva.

6. The suction device (1, 10, 20, 30) according to any of the preceding claims,
**characterized in that**
the probiotics (7, 17, 27, 37) are adhered to or immobilized on the outer surface of the suction device (1, 10, 20, 30) such that the probiotics (7, 17, 27, 37) are segregated by a nutrition (103).

7. The suction device (1, 10, 20, 30) according to any of the preceding claims,
**characterized in that**
the suction device (1, 10, 20, 30) is at least partially deformable, preferably at least the suction portion (3, 13, 23, 33) of the suction device (1, 10, 20, 30) is deformable, and the probiotics (7, 17, 27, 37) are adhered to or immobilized on the outer surface of the suction device (1, 10, 20, 30) such that the probiotics (7, 17, 27, 37) are mechanically segregated from the outer surface when the suction device (1, 10, 20, 30) is deformed.

8. The suction device (1, 10, 20, 30) according to any of the preceding claims,
**characterized in that**
the outer surface of the suction device (1, 10, 20, 30) is treated to enable adhesion of the probiotics (7, 17, 27, 37) .

9. The suction device (1, 10, 20, 30) according to any of the preceding claims,
**characterized in that**
the probiotics (7, 17, 27, 37) are mixed with a substance (8, 18, 28, 38) for promoting the adhesion or immobilization of the mix to/on the outer surface (6, 16, 26, 36) of the suction device (1, 10, 20, 30) and/or enhancing its stability.

10. The suction device (1, 10, 20, 30) according to claim 9,
**characterized in that**
the substance (8, 18, 28, 38) is a matrix, preferably comprising an oil, an emulsion or a gel.

11. The suction device (1, 10, 20, 30) according to claim 9 or 10,
**characterized in that**
the substance (8, 18, 28, 38) or matrix comprises carbohydrate or is based on or derived from carbohydrates.

12. The suction device (1, 10, 20, 30) according to claim 9 or 10,
**characterized in that**
the substance (8, 18, 28, 38) or matrix comprises proteinaceous materials or proteins.

13. The suction device (1, 10, 20, 30) according to claim 5 and 9 or 10,
**characterized in that**
said enzymes promote the dissolution of the substance (8, 18, 28, 38) or matrix by breaking bonds in said substance (8, 18, 28, 38) or matrix.

14. The suction device (1, 10, 20, 30) according to any of the preceding claims,
**characterized in that**
the suction device (1, 10, 20, 30), preferably at least the suction portion (3, 13, 23, 33) of the suction device (1, 10, 20, 30), is made of a flexible material, preferably a flexible polymeric material, more preferably silicon.

15. The suction device (1, 10, 20, 30) according to any of the preceding claims,
**characterized in that**
the suction device is a teat (10, 20, 30) or a pacifier (1).

16. The suction device (10, 20, 30) according to claim 15,
**characterized in that**
the suction device (10, 20, 30) has a hollow form and further comprises:
an inlet portion (12, 22, 32) having an inlet (14, 24) for entering nutrition (103), and
the suction portion (3, 13, 23, 33) is designed for sucking the nutrition (103) through the inlet (14, 24) into the suction device (10, 20, 30), wherein the suction portion (13, 23, 33) comprises at least one opening (15, 25, 35) for dispensing the sucked nutrition (103), wherein an inner surface (11, 21, 31) of the suction device (10, 20, 30) confines a flow path (P1, P2, P3) for the nutrition (103).

17. The suction device (1, 10, 20, 30) according to any of the preceding claims,
**characterized in that**
the suction device (1, 10, 20, 30) is enclosed in a protective shell, the protective shell preferably being air-tight and/or substantially oxygene tight and/or water tight, to isolate said suction device (1, 10, 20, 30) and protect the probiotics (7, 17, 27, 37) from degradation.

18. The suction device (1, 10, 20, 30) according to claim 16,
**characterized in that**
the protective shell comprises an aluminum foil or a polymeric material or a film or a combination thereof.

19. The suction device (1, 10, 20, 30) according to any of the preceding claims,
**characterized in that**
more than 10%, preferably more than 25%, preferably more than 50%, preferably more than 75%, preferably more than 90% of the outer surface of said suction device (1, 10, 20, 30) is covered by said probiotics (7, 17, 27, 37) and/or substance (8, 18, 28, 38) and/or matrix.

20. The suction device (1, 10, 20, 30) according to any of the preceding claims,
**characterized in that**
the segregation of the probiotics (7, 17, 27, 37) is enhanced by the contact, during suckling, of said nutrition (103) and said probiotics (7, 17, 27, 37) and/or said substance (8, 18, 28, 38) and/or said matrix.

21. A feeding apparatus (100) comprising:
a container (101) having an outlet (102), and
a suction device (10, 20, 30) according to any of the preceding claims, the suction device (10, 20, 30) being mounted on the outlet (102) of the container with its inlet portion (12, 22, 32).

22. The feeding apparatus according to claim 21,
**characterized in that**
the feeding apparatus (100) further comprises a fixing means (104) for removably linking the suction device (10, 20, 30) to the container (101).

23. The feeding apparatus according to claim 22,
**characterized in that**
the fixing means (104) is an adaptation ring with a closure mechanism or a screw thread.

24. A method for making the suction device (1, 10, 20, 30) according to any one of claims 1 to 20, said method comprising the step of contacting the outer surface of said suction device (1, 10, 20, 30) with said probiotics (7, 17, 27, 37) and/or said substance (8, 18, 28, 38) and/or said matrix for adhering or immobilizing the probiotics (7, 17, 27, 37) to/on the outer surface of the suction device (1, 10, 20, 30) such that the probiotics (7, 17, 27, 37) are segregated from the outer surface by external factors when the suction device (1, 10, 20, 30) is sucked.

25. The method according to claim 24, further comprising the step of evaporating an aquous portion of said probiotics (7, 17, 27, 37) and/or substance (8, 18, 28, 38) and/or matrix on said outer surface of the suction device (1, 10, 20, 30) for promoting said adhesion or immobilization.

26. The method according to claim 24 or 25, wherein said contacting is made in the form of spraying and/or dipping and/or depositing said probiotics (7, 17, 27, 37) and/or substance (8, 18, 28, 38) and/or matrix onto said outer surface of the suction device (1, 10, 20, 30).
